(19) Europäisches Patentamt
European Patent Office
Office européen des brevets

(11) Publication number: 0 154 455 B1

# (12) EUROPEAN PATENT SPECIFICATION

(45) Date of publication of patent specification: 02.05.90

(51) Int. Cl.[5]: C 08 G 59/40, C 07 C 243/34

(21) Application number: 85301177.3

(22) Date of filing: 22.02.85

## (54) Latent curing agent for epoxy resins.

(30) Priority: 25.02.84 JP 34867/84

(43) Date of publication of application:
11.09.85 Bulletin 85/37

(45) Publication of the grant of the patent:
02.05.90 Bulletin 90/18

(84) Designated Contracting States:
DE FR GB

(56) References cited:
EP-A-0 106 635
DE-C-1 054 704
US-A-3 456 006
US-A-3 467 707

(73) Proprietor: AJINOMOTO CO., INC.
5-8, Kyobashi 1-chome, Chuo-ku
Tokyo 104 (JP)

(72) Inventor: Hirai, Kiyomiki
No. 1155-2, Nakamaruko Nakahara-ku
Kawasaki-shi Kanagawa-ken (JP)
Inventor: Takeuchi, Koji
No. 806-40 Kamishirane-cho Asahi-ku
Yokohama-shi Kanagawa-ken (JP)
Inventor: Ito, Nobuo
No. 264-5, Ikusawa Oiso-machi
Naka-gun Kanagawa-ken (JP)
Inventor: Abe, Masahiro
No. 958, Kashimada Saiwai-ku
Kawasaki-shi Kanagawa-ken (JP)

(74) Representative: Bond, Bentley George et al
Haseltine Lake & Co. 28 Southampton Buildings
Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

The present invention relates to a latent curing agent for epoxy resins. More particularly, it relates to a latent curing agent for epoxy resins, which causes rapid resin curing at moderately elevated temperatures and which gives epoxy resin compositions having excellent storage stability at room temperature.

Epoxy resins are widely employed as electrical insulating materials, as various moulded products, and as adhesives and coatings, because they have excellent mechanical, electrical and chemical properties when cured with suitable curing agents, for example acid anhydride and amine curing agents. However, epoxy resin compositions containing amine curing agents cure rapidly at ordinary temperatures and at elevated temperatures and hence they lack storage stability. Also, epoxy resin compositions containing acid anhydride curing agents are stable at ordinary temperatures but heating for a long period of time at elevated temperatures is required for full curing. Usually, tertiary amines, quaternary ammonium compounds or organo metal complexes are further added to the compositions for the purpose of accelerating the curing rate. However, the addition of such cure accelerators impairs the storage stability markedly.

There are desired so-called latent curing agents which are compatible with epoxy resins to form compositions which are stable at relatively low temperatures and which rapidly cure when heated to elevated temperatures. Representative compounds which have been heretofore proposed as latent curing agents are dicyandiamide, dibasic acid hydrazides, boron trifluoride-amine adducts, guanamine and melamine. Among these compounds, dicyandiamide, dibasic acid hydrazides and guanamines are useful in formulating epoxy resin compositions having excellent storage stability, but full curing by means of these compounds can only be achieved by heating at a temperature higher than 150° for a long time. Boron trifluoride-amine adducts are not particularly useful owing to their high hygroscopic properties and their adverse effects upon the physical properties of the cured resins.

There has been heretofore known almost no latent epoxy curing agent which causes rapid curing at moderately elevated temperatures, that is at 100°C—150°C, and which gives epoxy resin compositions having excellent storage stability at ordinary temperatures. Epoxy resin compositions which contain latent curing agents are the so-called one pack-type epoxy resins.

One-pack type epoxy resins are preferable to conventional two-pack type epoxy resins because the former cannot be misformulated and can be used continuously. A need therefore continues to exist for an improved curing agent for a one-pack type epoxy resin.

The present invention provides a curing agent for one-pack epoxy resin compositions, which curing agent effectively cures the resin at low temperatures and which curing agent agent gives the composition superior storage properties, the curing agent being the hydrazide of the formula (I)

$$(NH_2NHCOCH_2CH_2)_2N(CH_2)_{11}CONHNH_2 \qquad (I)$$

This curing agent is useful in making storable one-package curable epoxy resin compositions. The curing agent, alone or together with other curing agent, can activate a rapid curing of epoxy resin compositions at relatively low temperatures and yet is extraordinarily resistant to gelling at 40°C for more than one month. The curing agent gives cured epoxy resins having excellent transparency and flexibility.

The hydrazide represented by the above general formula (I) is a novel compound which is not disclosed in the literature. It may be prepared by, for example, reacting an adduct of 1 mole of an ester of 12-aminododecanoic acid and 2 moles of an alkyl acrylate, with hydrazine hydrate, said adduct having the formula:

$$(R'OCOCH_2CH_2)_2N(CH_2)_{11}COOR \qquad (A)$$

wherein R and R' are alkyl groups.

The adduct (A) may be obtained by, for example, reacting 1 mole of an ester of 12-aminododecanoic acid with 2 moles of an alkyl acrylate in the presence of a basic catalyst such as potassium hydroxide and in the presence or absence of a solvent such as methanol. Methyl and ethyl acrylate are the preferred alkyl acrylates for reacting with the ester of 12-aminododecanoic acid.

The hydrazine of formula (I), in the form of white crystals, may be obtained by, for example, reacting 1 mole of adduct (A) with three or more moles of hydrazine hydrate in methanol for several hours. After filtration, the crystals may be recrystallized from methanol and dried.

Epoxy resin compositions containing the prescribed amount of the hitherto known dibasic hydrazides, such as adipic acid hydrazide, sebacic acid hydrazide, and isophthalic acid hydrazide, cure when heated to 150°C or higher temperatures. Contrary thereto, epoxy resin compositions containing the prescribed amounts of the hydrazide of the present invention have good storage stability and may be cured at 120°C to 140°C to give colourless, transparent and tough cured products.

The required amount of curing agent is determined by the number of active hydrogen atoms in the curing agent and by the number of epoxy atoms in the epoxy resin. In general, 0.5—1.5, preferably 0.7—1.2, active hydrogen equivalent weight per epoxy equivalent weight is employed.

As epoxy resins which may be used with the hydrazide curing agents of the present invention, various

well-known ones having an average of more than one epoxy group in the molecule may be employed. Representative epoxy resins are those based on glycidyl ethers of polyhydric phenols, especially the glycidyl ether of Bisphenol A, the glycidyl ether of Bisphenol F and the glycidyl ether of a phenolformaldehyde resin.

If desired, other curing agents, cure accelerators and fillers may be incorporated into the epoxy resin compositions of the present invention.

The following Example illustrates the invention.

Example

In a flask provided with cooling and mixing equipment, 101.5 g 0.47 mol) of 12-aminododecanoic acid, 600 ml of methanol and 60 g of concentrated hydrochloric acid were mixed. The mixture was heated for 4 hours with stirring. The solution was allowed to stand overnight to precipitate crystals, after concentration of the solution at room temperature. After filtration, the crystals were dried in vacuo to obtain 117.3 g (0.44 mol) of methyl 12-aminododecanolate hydrochloride salt.

39.1 g (0.147 mol) of the ester as the HCl salt were dissolved in methanol. After adding 63.3 g of methyl acrylate and 7.1 g of sodium hydrochloride, the solution was heated for 10 hours at 40°C. After the reaction, the reaction solution was concentrated, 50 ml of ethyl ether and 50 ml of water were added, the mixture was shaken for 10 minutes, and the ether and aqueous layers were separated. The ether layer was washed twice with 30 ml of water, and magnesium sulphate anhydride was added to dry it. The ether was then removed under reduced pressure to obtain 55.2 g (0.138 mol) of the following compound as a light yellowish liquid:

$$(CH_3OCOCH_2CH_2)_2N(CH_2)_{11}\ COOCH_3 \qquad (A')$$

The nuclear magnetic resonance (NMR) spectra (ppm) of (A') (in chloroform solvent, CDCl TMS standard) was as follows:

| | |
|---|---|
| 1.28 | (18H, $CH_2-(\underline{CH_2})_9\ CH_2$) |
| 2.2 – 2.5 | (8H, m, $N-\underline{CH_2}-(CH_2)_9$ <br> $(CH_2)_9\underline{CH_2}-COOCH_3$ <br> $N\underline{CH_2}CH_2COOCH_3$) |
| 2.74 | (4H, t, $NCH_2\underline{CH_2}COOCH_3$) |
| 3.65 | (9H, s, $\underline{CH_3}OCO-$) |

Then, 5.25 g of the ester A' were dissolved in 200 ml of methanol, 34.4 g hydrazine hydrate (as 80% aqueous solution) were added, and the solution was stirred for 8 hours at room temperature. After concentration of the solution, the white crystals which separated were removed by filtration. The crystals were washed with cold methanol, recrystallized from methanol, and dried in vacuo to obtain 40.5 g (0.101 mol) of crystals in the form of prisms. The analytical values of the product were as follows:

Melting point: 115, 117°C

Elemental analysis:

| | | C | H | N |
|---|---|---|---|---|
| found | (%) | 53.91 | 9.60 | 24.29 |
| calc'd for $C_{12}H_{20}N_6O_2$ | (%) | 51.41 | 7.19 | 29.98 |

NMR spectra (in water $D_2O$, DSS standard) δ (ppm)

| | |
|---|---|
| 1.29 | (18H, br, $-CH_2(\underline{CH_2})_9CH_2$) |
| 2.1 – 2.4 | (8H, m, $N-\underline{CH_2}(CH_2)_9$ <br> $(CH_2)_9\underline{CH_2}CONHNH_2$ <br> $NHCO-\underline{CH_2}CH_2N$) |
| 2.73 | (4H, t, $NHCO-CH_2\underline{CH_2}N$) |

The reactivity and storage stability of epoxy resin compositions were evaluated. The compounds of the compositions are given in Table 1. The components were mixed in a mortar to prepare the compositions.

The reactivity of the compositions was determined by measuring the onset temperature and peak temperature by differential thermal analysis (DTA) under the following conditions:

Composition weight: about 10 mg

Standard material: α-$Al_2O_3$

Heating rate: 5°C/min.

The reactivity of the compositions was also determined by putting the compositions into a Geer's oven for 60 minutes and measuring the cure temperature.

The storage stability of the compositions was determined by putting the compositions into a Geer's oven set at 40°C, the day upon which the composition became non-fluid being measured.

The results obtained, as given in Table 2, show that the latent curing agent of this invention gives compositions having an excellent storage stability, and has good reactivity. In particular, the reactivity of the agent is superior to that of the control agent.

Table 1

| | | Formulation no. | | | |
|---|---|---|---|---|---|
| | | 1 | 2 | 3 | 4 |
| The present invention | Epon 828* | 100 | 100 | 100 | 100 |
| | Compound | 3 | | | |
| | Aerosil** | 3 | | | |
| Control | Adipic dihydrazide | | 23 | | |
| | Isophthalic dihydrazide | | | 26 | |
| | Dicyandiamide | | | | 8 |

* A product of Shell Chemical Co. (bisphenol A type epoxy resin having epoxy equivalent of 175 - 120)

** A product of Japan Aerosil Co. (ultra microsphere silica anhydride)

Table 2

| | Formulation No. | Reactivity | | | |
|---|---|---|---|---|---|
| | | Onset temp. (°C) | Peak temp. (°C) | Cure condition (120°C, 60 min) | Storage stability (40°C) |
| The Present invention | No. 1 | 100 | 138 | cured non-colored | 4 weeks |
| Control . | No. 2 | 151 | 173 | Not cured | 4 weeks |
| | No. 3 | 158 | 192 | " | " |
| | No. 4 | 160 | 199 | " | " (Partial separation occured) |

**Claims**

1. The compound having the formula:

$$(NH_2NHCOCH_2CH_2)_2N(CH_2)_{11}CONHNH_2.$$

2. A curing agent suitable for use with epoxy resin compositions, comprising the compound claimed in claim 1.

3. A curable epoxy resin composition comprising (a) an epoxy resin having an average of more than one epoxy group per molecule and (b), as a curing agent, the compound claimed in claim 1.

4. A composition as claimed in claim 3, wherein the amount of said compound is sufficient to provide 0.5—1.5 times the active hydrogen equivalent weight based on the epoxy equivalent weight of the epoxy resin.

5. A composition as claimed in claim 3 or 4, wherein said epoxy resin is a polyglycidyl ether of a polyhydric phenol.

6. A cured resin obtained by contacting an epoxy resin having an average of more than one epoxy group per molecule with, as a curing agent, the compound claimed in claim 1 in an amount sufficient to provide 0.5—1.5 times the active hydrogen equivalent weight based on the epoxy equivalent weight of the epoxy resin.

**Patentansprüche**

1. Verbindung der Formel:

$$(NH_2NHCOCH_2CH_2)_2N(CH_2)_{11}CONHNH_2.$$

2. Zur Verwendung mit Epoxyharz-Zusammensetzungen geeignetes Härtungsmittel, welches die Verbindung gemäß Anspruch 1 umfaßt.

3. Härtbare Epoxyharz-Zusammensetzung, enthaltend (a) ein Epoxyharz mit durchschnittlich mehr als einer Epoxygruppe pro Molekül und (b) als Härtungsmittel die Verbindung gemäß Anspruch 1.

4. Zusammensetzung gemäß Anspruch 3, in der die Menge dieser Verbindung ausreicht, um das 0,5- bis 1,5-fache Äquivalentgewicht an aktivem Wasserstoff, bezogen auf das Epoxyäquivalentgewicht des Epoxyharzes, zu liefern.

5. Zusammensetzung gemäß Anspruch 3 oder 4, wobei das Epoxyharz ein Polyglycidylether eines mehrwertigen Phenols ist.

6. Gehärtetes Harz, das erhalten wird, indem ein Epoxyharz mit durchschnittlich mehr als einer Epoxygruppe pro Molekül mit der Verbindung gemäß Anspruch 1 als Härtungsmittel in einer Menge, die ausreicht, um das 0.5- bis 1,5-fache des aktiven Wasserstoffäquivalentgewichts, bezogen auf das Epoxyäquivalentgewicht des Epoxyharzes, zu liefern, in Kontakt gebracht wird.

**Revendications**

1. Le composé répondant à la formula

$$(NH_2NHCOCH_2CH_2)_2N(CH_2)_{11}CONHNH_2.$$

2. Un agent durcisseur adapté pour l'utilisation avec des compositions de résines époxydes, comprenant le composé selon la revendication 1.

3. Une composition durcissable de résines époxydes comprenant (a) une résine époxyde ayant une moyenne de plus d'un groupe époxy par molécule et (b) comme agent durcisseur le composé selon la revendication 1.

4. Une composition selon la revendication 3, dans laquelle la quantité dudit composé est suffisante pour donner 0,5—1,5 fois le poids équivalent d'hydrogène actif par rapport au poids équivalent d'époxyde de la résine époxyde.

5. Une composition selon la revendication 3 ou 4, dans laquelle ladite résine époxyde est un éther polyglycidylique d'un polyphénol.

6. Une résine durcie obtenue en mettant en contact une résine époxyde ayant une moyenne de plus d'un groupe époxy par molécule avec, comme agent durcisseur, le composé selon la revendication 1 en quantité suffisante pour donner 0,5—1,5 fois le poids équivalent d'hydrogène actif, par rapport au poids équivalent d'époxyde de la résine époxyde.